# EUROPEAN PATENT APPLICATION

(11) **EP 0 709 099 A2**
(43) Date of publication of application: **01.05.1996**
(21) Application number: 95114715.6
(22) Date of filing: 19.09.1995
(51) Int. Cl.: A61K 47/48, A61K 9/08

(54) **An aqueous nasal suspension comprising cyclodextrin**

(30) Priority: 28.09.1994 JP 233267/94
(71) Applicant: Senju Pharmaceutical Co., Ltd., Osaka-shi, Osaka 541 (JP)
(72) Inventor: Kimura, Masako, Kakogawa, Hyogo 675 (JP); Morita, Yasushi, Toyonaka 560 (JP); Fukushi, Kunihiro, Suita 565 (JP)
(74) Representative: von Kreisler, Alek, Dipl.-Chem.

(57) **Abstract**

This invention relates to an aqueous nasal suspension comprising a cyclodextrin and hardly soluble drug whose one part by weight requires 1000 or more parts by weight of water to yield a homogeneous mixture at 25°C under one atmospheric pressure and whose stability constant in relation to the cyclodextrin calculated by solubility method is not greater than 1000.

The aqueous nasal suspension of this invention enhances the pharmacological efficacy of a hardly soluble drug in water, improves its retention in nasal mucosa, and prolongs the action of the drug so that the frequency of administration can be decreased. Therefore, this nasal suspension can be used as a meritorious topical nasal preparation assuring improved patient compliance.

## Description

### Field of the Invention

This invention relates to aqueous nasal suspensions of hardly soluble drugs. More particularly this invention relates to an aqueous nasal suspension comprising a cyclodextrin and a hardly soluble drug which has a stability constant of not greater than 1000 in relation to the cyclodextrin.

### Background of the Invention

A nasal drug preparation is generally administered in the form of a water-based liquid or a powder, but the drug so administered flows down into the throat in a comparatively short time after administration or becomes intermingled with the nasal secretion and excreted spontaneously from the nostrils. When the drug is administered in a powdery form and the particle size of the powder is not greater than 10µ m, the particles find their way into the trachea, while particles of greater than 500 µm in diameter settle down on the non-villous anterior part of the nasal cavity. Therefore, in order that a nasal drug preparation may exhibit its efficacy, it must be administered often. However, as frequent administration of a topical nasal reparation causes a chronic edematous lesion of the nasal mucosa which is called drug rhinitis, development of a nasal preparation assuring good nasal mucosal retention of the drug and prolonged drug action has been desired.

Meanwhile, cyclodextrins are cyclic oligosaccharides having the property to entrap a variety of compounds in their hydrophobic cage like structures to form water-soluble complexes called inclusion complexes and, as such, have been used for the purpose of enhancing the solubility or stability of drugs. Thus, cyclodextrins are used in the production of parenteral products or ophthalmic and other liquid preparations of hardly soluble or practically insoluble drugs. It is also known that the drug included in a cyclodextrin molecule is gradually released from the inclusion site, so that a prolongation of drug action can be expected. Cyclodextrin occurs as α-, β-, and γ-compounds which have their own unique characteristics but as far as the solubilization or stabilization of hardly soluble or practically insoluble drug is concerned, most studies so far undertaken are concerned with β- and γ-cyclodextrins, inclusive of their derivatives, and a number of reports are available on the subject (e.g. JP Publication 43314/94, JP Publication 70612/93, JP Kokai Publication 11018/91, JP Kokai Publication 178765/93, JP Kokai Publication 134093/92 and JP Kokai Publication 167228/90).

With regard to nasal preparations utilizing cyclodextrins, there is a report on a pharmaceutical product prepared by entrapping a hardly soluble or practically insoluble antiviral drug in the cage like structure of a cyclodextrin by taking advantage of that unique property of a cyclodextrin (JP Kokai Publication 45319/89) as well as a report on a trans nasal drug delivery system comprising a polypeptide, which is hardly absorbed from the gastrointestinal tract, and a cyclodextrin (JP Publication 19092/90).

However, a hardly soluble drug which has a stability constant of not greater than 1000 in relation to a cyclodextrin calculated by solubility method does not lend itself well to said solubilization or stabilization with a cyclodextrin. Therefore, the utility potential of any pharmaceutical composition and, for that matter, a nasal preparation, which comprises a cyclodextrin and a drug has not been investigated.

### Summary of the Invention

The inventors found surprisingly that when a hardly soluble drug which has a low stability constant in relation to the cyclodextrin calculated by solubility method is suspended in an aqueous solution of the cyclodextrin, the mucosal retention time of the drug is remarkably prolonged and pharmacological action of the drug is unexpectedly increased. This invention has been developed on the basis of the above finding.

This invention, therefore, relates to a nasal suspension of a hardly soluble (inclusive of "very slightly soluble", "practically insoluble and insoluble" in water as indicated by the descriptive terms in USP 23, page 2071) drug which has a stability constant calculated by the solubility method of not greater than 1000 in relation to the cyclodextrin in an aqueous solution of the cyclodextrin.

### Detailed Description of the Invention

The cyclodextrin that can be used in this invention includes α - cyclodextrin, β-cyclodextrin, and γ-cyclodextrin, inclusive of their derivatives such as dimethyl-β-cyclodextrin, hydroxypropyl-γ-cyclodextrin and so on. Among these cyclodextrin species, α-cyclodextrin which has low stability constants and low inclusion ratios in relation to various drugs is particularly preferred.

Such a cyclodextrin is used as dissolved in water at a concentration of generally 1.0-10.0 w/v% or preferably 2.5-7.5 w/v% and two or more different cyclodextrins can be used in combination depending on the specific objective.

The hardly soluble drug for use in this invention requires 1000 or more parts by weight of water to yield a homogeneous mixture with one part by weight of said drug at 25°C under one atmospheric pressure, preferably 10000 or more, and more preferably 100000 or more parts by weight of water.

The drug also shows a stability constant in relation to cyclodextrin calculated by the solubility method of generally not greater than 1000, preferably not greater than 500, and more preferably not greater than 300.

The term 'stability constant' as used throughout this specification is an indicator of stability of a cyclodextrin-drug complex and represents the degree in which such a complex is formed between a cyclodextrin and a drug, too.

The stability constant of the drug in relation to cyclodextrin calculated by the solubility method is determined in the manner that, with the concentration of each drug being kept unchanged, a mixture of the drug and a cyclodextrin solution of varying concentration is shaken on a shaker at the rate of 200 cycles/min at 25°C for 24 hours to construct a solubility phase diagram of the drug at saturation in cyclodextrin solution and the stability constant is calculate by the method of Higuchi et al. [Advances in Analytical Chemistry and Instrumentation, Vol. 4, 144-148 (1965)].

The drug may be selected from among steroidal antiinflammatory drugs, non steroidal antiinflammatory drugs, anti-histaminic drugs, antiallergic drugs, antimicrobial drugs and so on.

Among such hardly soluble drugs, loteprednol etabonate, fluorometholone, hydrocortisone, beclometasone dipropionate, fluticasone propionate and betamethasone can be mentioned as typical steroidal antiinflammatory drugs. Mefenamic acid, etc. may be mentioned as examples of the non steroidal antiinflammatory drug. The antihistaminic drug is exemplified by clemastine fumarate and terfenadine. Tranilast, etc. can be mentioned as examples of the antiallergic drug. Among said antimicrobial drugs are antibiotics such as erythromycin, tetracycline, etc., and chemotherapeutic drugs such as sulfamethizole, sulfamethoxazole, sulfisoxazole and so on.

The concentration of the hardly soluble drug in the present nasal suspension is generally 0.01 to 10.0 w/v%, preferably 0.05-5.0 w/v% and more preferably 0.1 - 1.0 w/v%.

These hardly soluble drugs can be used singly or in combination according to the specific objective of use of the nasal preparation.

The aqueous nasal suspension of this invention is preferably put to use with the particle size of the drug selected from the range conventionally used for nasal application. The particle diameter is generally 1.0-100µ m and preferably 5.0-50µ m.

While the formulating ratio of a cyclodextrin to the drug is selected according to the stability constant of the drug in relation to the cyclodextrin, the cyclodextrin is used in a proportion of generally 1.0-10.0 mols and preferably 2.5-7.5 mols to each mol of the drug. Specifically when α-cyclodextrin is used, for instance, it is preferable to formulate α-cyclodextrin in a proportion of generally 1.0-10.0 mols or preferably 2.5-7.5 mols per mol of a drug with a stability constant of not greater than 200 in relation to α-cyclodextrin.

The aqueous nasal suspension of this invention may be supplemented with various additives which are conventionally used in known nasal drugs. Among such additives are preservatives, isotonizing agents, buffers, stabilizers, pH control agent, thickeners, and suspending agents. The preservative that can be used includes parabens (e.g. methyl p-hydroxybenzoate, propyl p-hydroxybenzoate, etc.), invert soaps (e.g. benzalkonium chloride, benzethonium chloride, chlorhexidine gluconate, cetylpyridinium chloride, etc.), alcohol derivatives (e.g. phenethyl alcohol, benzyl alcohol etc.), organic acids and salts thereof (e.g. sodium dehydroacetate, sorbic acid and salts thereof), phenols (e.g. p-chloromethoxyphenol, p-chloro-m-cresol, etc.), and organomercury compounds (e.g. thimerosal, phenylmercuric nitrate, nitromersol, etc.), among others. The isotonizing agent that can be used includes sodium chloride, sorbitol, mannitol, glycerin and so on. The buffer includes boric acid and its salts, phosphoric acid salts, acetic acid salts, amino acid salts, and so on. The stabilizer that can be used includes anti oxidants (e.g. sodium sulfite, sodium hydrogensulfite, sodium hydrogen metasulfite, etc.) and chelating agents (e.g. sodium edetate, citric acid and its salts). The pH control agent includes hydrochloric acid, acetic acid, sodium hydroxide, phosphoric acid, and so on. The thickener includes polyhydric alcohols (glycerin, macrogols, etc.), saccharides (sorbitol, mannitol, sucrose, etc.), cellulose derivatives, (methylcellulose, carboxymethylcellulose sodium, hydroxypropylmethylcellulose, etc.) and synthetic polymers (polyvinyl alcohol, polyvinylpyrrolidone, carboxyvinyl polymer, etc.). The suspending agent includes the above-mentioned cellulose derivatives and synthetic polymers as well as surfactants (cationic surfactants such as quaternary ammonium salts, anionic surfactants such as alkylsulfuric acid salts, and amphoteric surfactants such as lecithin), among others.

While the amounts of such additives should be selected according to the active ingredient used and its amount, the composition is preferably formulated to approximate the physiological status (isotonic to the nasal secretion). Thus, the osmotic pressure, for example, of the composition should be controlled within the range corresponding to that of 0.2-4.0 w/v% saline, preferably 0.5-2.0 w/v% saline and, for still better results, 0.9-1.5 w/v% of saline.

The aqueous nasal suspension of this invention is advantageously put to use as adjusted to the pH range generally used for nasal application and generally to pH 5-7.

The aqueous nasal suspension of this invention is advantageously put to use as adjusted to the osmotic pressure range generally used for nasal application and generally to 140-1140 mOsm, preferably to 200-870 mOsm, and for still better results to 280-310 mOsm.

The aqueous nasal suspension of this invention is advantageously put to use as adjusted to a viscosity within the range generally used for nasal application and generally to 800 cSt or less, and preferably 600 cSt or less.

The aqueous nasal suspension of this invention may be further supplemented with other medicinal substances such as a vasoconstrictor, a surface anesthetic, and so on. The vasoconstrictor that can be used includes naphazoline nitrate and phenylephrine hydrochloride, among others. The surface anesthetic includes lidocaine hydrochloride, mepivacaine hydrochloride and bupivacaine hydrochloride, among others.

The aqueous nasal suspension of this invention can be manufactured by the per se known technology. For example, it can be manufactured by the procedure described in Formulation Guidelines, 9th Revised Edition (Ed. by Japanese Association of Pharmacists, pp. 128-129, Yakuji Nippo Sha).

The aqueous nasal suspension of this invention as manufactured in the above manner increases and prolongs the pharmacological efficacy of the hardly soluble drug to help reduce the instillation frequency and, hence, improve patient compliance. The aqueous nasal suspension of this invention can be used in the same ways as the known topical nasal drugs in general, for example by spraying or dripping. When the aqueous nasal suspension of this invention is administered by the spray method in the treatment of allergic rhinitis or vasomotor rhinitis in adults, the concentration of beclomethasone dipropionate is controlled, although it depends on age, body weight, and clinical condition, generally within range of 0.05-0.5 w/v%, preferably 0.1-0.25 w/v%, and a mist of the preparation is sniffed from a sprayer of a nebulizer in a dose of 1-2 puffs 1-2 times daily. When the dripping method is employed in allergic rhinitis, the nasal suspension of this invention can be administered at a loteprednol etabonate concentration of 0.1-1.0 w/v%, preferably 0.25-0.5 w/v%, although the dosage depends on age, body weight, clinical condition, etc., in a dose of 1-2 drops 1-2 times a day from the nostril with the patient in standing or sitting posture with the neck bent backward.

The following examples and test example are intended to describe the invention in further detail and demonstrate the effectiveness of this invention, it being to be understood that these are merely illustrative and not limitative of the scope of the invention.

### Example 1

| Loteprednol etabonate-containing nasal suspension | |
|---|---|
| Loteprednol etabonate | 0.5 g |
| α-Cyclodextrin | 5.0 g |
| Sodium chloride | 0.9 g |
| Sodium acetate | 0.1 g |
| Sodium edetate | 0.02 g |
| Benzalkonium chloride | 0.005 g |
| Sterilized pure water | 100.0 ml |

The above formulation was stirred overnight and adjusted to pH 5.0 with hydrochloric acid.

### Example 2

| Loteprednol etabonate-containing nasal suspension | |
|---|---|
| Loteprednol etabonate | 0.5 g |
| β-Cyclodextrin | 1.8 g |
| Sodium chloride | 0.9 g |
| Sodium acetate | 0.1 g |
| Sodium edetate | 0.02 g |
| Benzalkonium chloride | 0.005 g |
| Sterilized pure water | 100.0 ml |

The above formulation was stirred overnight and adjusted to pH 5.0 with hydrochloric acid.

### Example 3

| Fluorometholone-containing nasal suspension | |
|---|---|
| Fluorometholone | 0.1 g |
| γ-Cyclodextrin | 4.0 g |
| Sodium chloride | 0.8 g |
| Sodium dihydrogen phosphate | 0.1 g |
| Benzalkonium chloride | 0.01 g |
| Sterilized pure water | 100.0 ml |

The above formulation was stirred overnight and adjusted to pH 7.0 with sodium hydroxide.

### Example 4

| Tranilast-containing nasal suspension | |
|---|---|
| Tranilast | 0.1 g |
| α-Cyclodextrin | 4.0 g |
| Sodium chloride | 0.8 g |
| Sodium dihydrogen phosphate | 0.1 g |
| Benzalkonium chloride | 0.01 g |
| Sterilized pure water | 100.0 ml |

The above formulation was stirred overnight and adjusted to pH 7.0 with sodium hydroxide.

### Example 5

| Beclometasone dipropionate-containing nasal suspension | |
|---|---|
| Beclometasone dipropionate | 0.5 g |
| α-Cyclodextrin | 5.0 g |
| Sodium chloride | 0.8 g |
| Sodium dihydrogen phosphate | 0.1 g |
| Benzalkonium chloride | 0.01 g |
| Sterilized pure water | 100.0 ml |

The above formulation was stirred overnight and adjusted to pH 6.3 with hydrochloric acid.

### Example 6

| Mefenamic acid-containing nasal suspension | |
|---|---|
| Mefenamic acid | 0.5 g |
| α-Cyclodextrin | 5.0 g |
| Sodium chloride | 0.8 g |
| Sodium acetate | 0.1 g |
| Benzalkonium chloride | 0.005 g |
| Sterilized pure water | 100.0 ml |

The above formulation was stirred overnight and adjusted to pH 5.8 with hydrochloric acid.

### Test Example 1

### Stability constant of drugs

The stability constant of the drug in relation to cyclodextrin was calculated applying the formula (8) on page 146, in "Advances in Analytical Chemistry and Instrumentation", Vol. 4, (1965). The results are shown in Table 1.

**Table 1**

| Stability Constant of the Drug in relation to Cyclodextrin | | |
|---|---|---|
| Drug | Stability Constant | |
| | α-CyD | β-CyD |
| Loteprednol Etabonate | 114 | 305 |
| Fluorometholone | 54 | 911 |

### Test Example 2

### Effect on the Rat Rhinitis Model

### Method

Using male Wistar rats (body weights ca. 170 g, 7 or 10 animals/group) under pentobarbital anesthesia, 3µ l of 0.5 v/v% acetic acid was dripped into both nostrils. After 20 minutes, 3µ l of 10 w/v% arachidonic acid was similarly administered to induce a nasal mucosal inflammation. Four hours after induction of inflammation, the trachea was incised and cannulated for securing an airway. Then, the esophagus was incised and an elastor tube was inserted from the neck region up to the nasal cavity. Immediately thereafter, 1 ml of 1 w/v% Evans blue was injected intravenously and the nasal cavity was perfused with 5 w/v% formamide-containing saline from the elastor tube at a flow rate of 10 ml/hr for 6 minutes. The perfusate was collected from both nostrils. The absorbance of the collected perfusate was measured at 625 nm.

As test drugs, 10µ l each of the nasal suspension of Example 1 (LTP/α-CyD nasal suspension) and the nasal suspension after elimination of α-cyclodextrin from Example 1 (LTP nasal suspension) were respectively administered into both nostrils under pentobarbital anesthesia 1 hour or 15 hours before nasal administration of 0.5 v/v% acetic acid. The control group was nasally dosed with saline in lieu of test drugs.

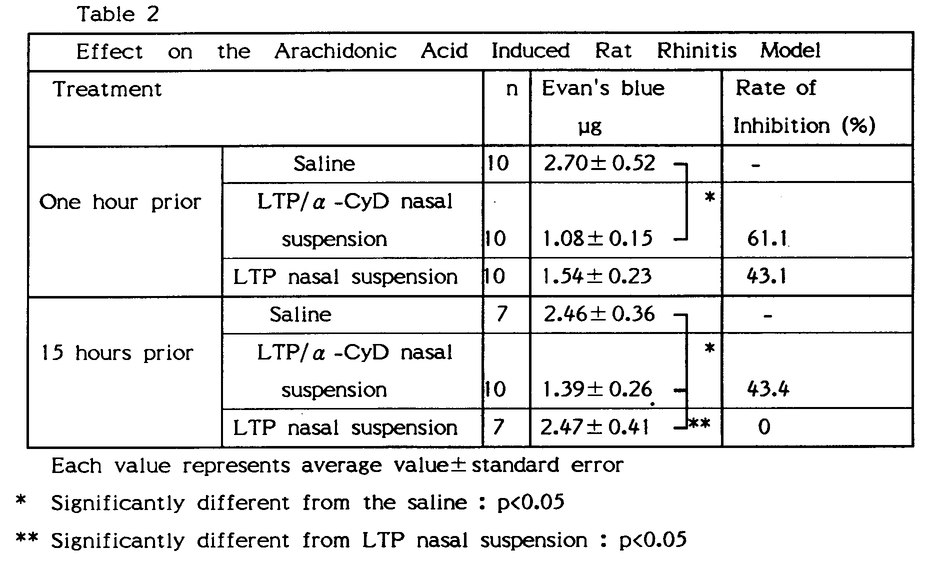

### Results

The results are shown in Table 2. In the case where the LTP/α-CyD nasal suspension was administered one hour before induction of rhinitis, the dye leakage from the nasal mucosa was significantly inhibited in the LTP/α-CyD nasal suspension treatment group. This inhibitory effect was also found in the case where the nasal preparation was administered 15 hours before induction of inflammation. However, in the LTP nasal suspension treatment group, an inhibitory effect was noted in the case where nasal administration was performed one hour before induction of rhinitis but this effect was not significant. Moreover, when the nasal administration was performed 15 hours before induction of inflammation, no effect was found. These results indicated that formulating loteprednol etabonate with α-cyclodextrin potentiated and prolonged its antiinflammatory action.

### Rat Nasal Mucosal Retention Test

### Method

Using male Wistar rats (body weights ca. 170 g, 5 animals/group) under pentobarbital anesthesia, 10µ l of each test suspension was administered into both nostrils. At 0.5, 1, 2, 4, 6, 8, 12, and 24 hours after administration, the whole blood was drawn from the abdominal aorta of the rats under pentobarbital anesthesia and, at the same time, the nasal mucosa was collected. After the wet weight of the nasal mucosa was determined, loteprednol etabonate was extracted with ethyl acetate. The loteprednol etabonate in the nasal mucosa was assayed by high performance liquid chromatography. As test drugs, the nasal preparation of Example 1 (LTP/α-CyD nasal suspension) and the nasal preparation prepared by elimination of α-cyclodextrin from the prerparation of Example 1 (LTP nasal suspension) were used.

### Results

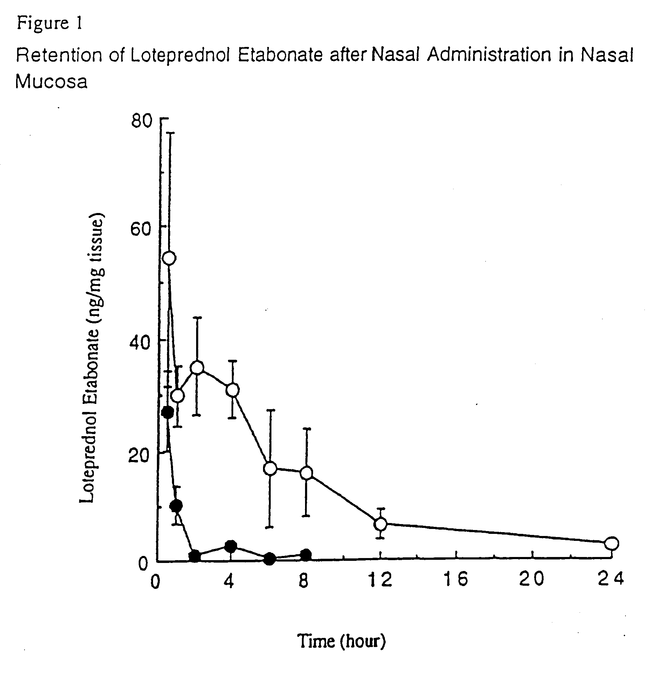

The results are shown in Figure 1. The rate of elimination of loteprednol etabonate from nasal mucosa and the area under the concentration curve (AUC) of the drug in nasal mucosa, as determined from the data plotted in Figure 1, are shown in Table 3. The rate of elimination of loteprednol etabonate in the LTP/α-CyD nasal suspension treatment group was 1/20 times as slow as that in the LTP nasal suspension treatment group and the AUC in the LTP/α-CyD nasal suspension treatment group was about 10 times as large as that in the LTP nasal suspension treatment group, indicating that the nasal suspension prepared by adding α-cyclodextrin to a suspension of loteprednol etabonate is better retained in the nasal mucosa.

The rate of elimination of LTP is calculated by the following formula:
Elimination Rate (hr⁻¹)= ln C₁- ln C₂/ t₁-t₂
t₁,t₂ : Time lapse (hr) after administration of LTP
C₁,C₂ : Respective LTP Concentrations of nasal mucosa at t₁ and t₂

**Table 3**

| Improved Retention of Loteprednol Etabonate in Nasal Mucosa | | | |
|---|---|---|---|
| Treatment | Rate of elimination (hr⁻1) | AUC [(ng/mg-tissue)×hr] | Cmax (ng/mg-tissue) |
| LTP/α-CyD nasal suspension | 0.12 | 314.1 | 55.4 |
| LTP nasal suspension | 2.26 | 29.0 | 27.2 |

### Brife Description of the Drawing

### Figure 1

Retention of Loteprednol Etabonate after Nasal Administration in Nasal Mucosa.

Each graph shows concentration of loteprednol etabonate in rat nasal mucosa after nasal administration. ○ represents the LTP/α-CyD nasal suspension treatment group and ● represents the LTP nasal suspension treatment group.

## Claims

1. An aqueous nasal suspension which comprises a cyclodextrin and a hardly soluble drug whose one part by weight requires 1000 or more parts by weight of water to yield a homogeneous mixture at 25°C under one atmospheric pressure and whose stability constant in relation to the cyclodextrin calculated by solubility method is not greater than 1000.

2. The aqueous nasal suspension according to Claim 1 wherein said hardly soluble drug is for topical use.

3. The aqueous nasal suspension according to Claim 1 wherein said hardly soluble drug is one or more species selected from the group consisting of steroidal antiinflammatory, non steroidal antiinflammatory, antihistaminic, antimicrobial and antiallergic drugs.

4. The aqueous nasal suspension according to Claim 3 wherein said hardly soluble drug is one or more species selected from among loteprednol etabonate and fluorometholone, beclometasone dipropionate and fluticasone propionate for said steroidal antiinflammatory drug, mefenamic acid for said non steroidal antiinflammatory drug, clemastine fumarate and terfenadine for said antihistaminic drug, and tranilast for antiallergic drug.

5. The aqueous nasal suspension according to Claim 4 wherein said steroidal antiinflammatory drug is loteprednol etabonate.

6. The aqueous nasal suspension according to Claim 1 wherein a cyclodextrin is contained at a concentration of 1.0-10.0 w/v%.

7. The aqueous nasal suspension according to Claim 1 wherein said cyclodextrin is α-cyclodextrin.
